# EUROPEAN PATENT SPECIFICATION

(11) **EP 3 235 791 B1**
(45) Date of publication and mention of the grant of the patent: **07.06.2023**
(21) Application number: 17166806.4
(22) Date of filing: 18.04.2017
(51) Int. Cl.: C02F 3/28, C07C 37/00, C02F 3/34, C02F 103/32, C02F 101/34

(54) **PROCESS FOR TREATING OLIVE MILL WASTE WATERS**
VERFAHREN ZUR BEHANDLUNG VON ABWÄSSERN AUS OLIVENMÜHLEN
PROCÉDÉ DE TRAITEMENT D'EAUX USÉES DE MOULINS À OLIVES

(30) Priority: 18.04.2016 IT UA20162673
(43) Date of publication of application: 25.10.2017
(73) Proprietor: Bisarcha S.r.l., 56122 Pisa (IT)
(72) Inventor: CECCHI, Antonio, 56122 PISA (IT); FRANCESCHI, Massimiliano, 57124 LIVORNO (IT); BAZZICHI, Agostino, 56019 VECCHIANO (IT)
(74) Representative: Cattaneo, Elisabetta

(56) References cited:
- EP-A1- 1 870 171
- WO-A1-2008/090460
- GB-A- 2 415 136
- JINGREN HE ET AL: "Stability and antioxidant potential of purified olive mill wastewater extracts", FOOD CHEMISTRY, ELSEVIER LTD, NL, vol. 131, no. 4, 26 September 2011 (2011-09-26), pages 1312-1321, XP028118850, ISSN: 0308-8146, DOI: 10.1016/J.FOODCHEM.2011.09.124 [retrieved on 2011-10-04]
- Mongi Feki ET AL: "Effect of storage of olive mill wastewaters on hydroxytyrosol concentration", European Journal of Lipid Science and Technology, 1 January 2006 (2006-01-01), pages 1021-1027, XP055589018, Retrieved from the Internet: URL:https://onlinelibrary.wiley.com/doi/ep df/10.1002/ejlt.200500348 [retrieved on 2019-05-15]

## Description

### FIELD OF THE INVENTION

The invention relates to a process for treating olive mill waste waters, particularly for the enrichment of olive mill waste waters with phenol and polyphenol compounds.

### PRIOR ART

Olive mill waste waters (OMWW) are aqueous solutions of organic and mineral substances, also containing suspended vegetable solid material resulting from the separation of oil wort. Their composition is extremely variable and depends on several factors, including the type of olive cultivar and their degree of ripening, the soil, the extractive process and the preservation method.

Olive mill waste waters comprise several ingredients, including antioxidant substances such as polyphenols, which are of great interest in the dietary-nutritional, food and pharmaceutical sectors.

A typical composition of olive mill waste waters is shown in the following Table 1, while Table 2 shows the values of some important chemical-biological parameters.

**Table 1. Typical OMWW composition.**

| | |
|---|---|
| Organic substances (%) | 3-16 |
| Nitrogenous substances (%) | 1.2-2.4 |
| Sugars (%) | 1-8 |
| Minerals (%) | 0.5-1.4 |
| Total polyphenols (%) | 0.34-1.13 |
| N (%) | 0.5-1.5 |
| P (%) | 0.03-0.11 |
| K (%) | 0.27-0.72 |
| Ca (%) | 0.012-0.075 |
| Mg (%) | 0.01-0.04 |
| Na (%) | 0.004-0.09 |
| Total solids (%) | 4.5-17.4 |

**Table 2. Some chemical-biological parameters of OMWW**

| | |
|---|---|
| pH | 4-6 |
| Electrical conductivity at 25 °C (mS/cm) | 8-20 |
| COD (mg O₂/L) | 40000-195000 |
| BOD₅ (mg O₂/L) | 35000-100000 |

The production and the features of olive mill waste waters are directly linked to the production of oil olives and their features, as well as to the used extractive process. Generally, for 100 kg of olives, 70-100 kg of olive mill waste waters are obtained. The olive mill waste waters are characterized by a color that can range from green to dark brown, depending on the storage conditions during which various fermentation processes take place, and are characterized by a typical, rather intense odor, similar to that of the stone fruit from which they are derived.

The pH value, typically between 4-6, is determined by the content of organic acids such as malic, citric, tartaric, succinic and oxalic acid. These pH oscillations are attributable to the variety, the ripening period and the storage duration of olives, while in general the pH is poorly influenced by the selected extraction system. The organic fraction contains sugars, tannins, phenolic compounds, polyalcohols, pectins, lipids and nitrogenous substances.

Sugars are the organic substances that prevail, especially fermentable sugars such as glucose (70%), mannitol (14%), fructose (10%), sucrose (5%), galactose (1%) and cellulose.

As far as the nitrogenous component is concerned, all amino acids, in particular glutamic acid and proline, were found.

It is the organic fraction that determines the high polluting power of olive mill waste waters, which are normally characterized by very high chemical oxygen demand (COD) and biological oxygen demand (BOD5), respectively ranging between 90 grams O₂/L and 30 grams O₂/L or waters originated from the most modern centrifugal plants, and even 150 grams O₂/L and 90 grams O₂/L for waters from traditional plants.

Because of its very high pollutant potential, it is therefore clear that olive mill waste waters are nowadays waste to dispose of.

Currently, it is disposed of as non-hazardous waste or by spreading on farmland. In recent years, however, much has been attempted to regenerate the olive mill waste waters by converting them from waste to dispose of to a reusable product, in order to attain, at the same time, the exploitation of this waste water and the reduction of its environmental impact.

Since olive mill waste waters contain phenolic compounds with significant antioxidant properties, it has been suggested to use them as a source of these compounds.

The phenolic compounds present in olive mill water waters, overall at concentrations of the order of 3 to 10 g/L, are molecules both with low and high molecular weight. Among the low PM phenols, for example, hydroxytyrosol (HT), tyrosol (TY), catechol, methylcatechol, oleuropein, caffeic, gallic, vanillic and coumaric acid are identified, which have extraordinary antioxidant properties, with positive effects on the coronary heart disease (Chd) apparatus and antitumor properties (breast, colon, prostate).

It is also known that hydroxytyrosol can account for up to 50% of the total polyphenols of the olive mill waste waters and has been recognized as one of the most powerful antioxidants in nature. The ORAC index for hydroxytyrosol is 40,000 µmolTE/g, which is about ten times greater than green tea and at least twice that of CoQ10.

It is also known that another factor influencing the chemical-physical properties of olive mill waste waters and therefore the concentration of the phenolic compounds is their degree of "ripening"; in relation to the storage method and duration, due to biological processes carried out by some yeasts and bacteria and in the presence of organic acids (mainly malic and citric), olive mill waste waters undergo a pH reduction up to values around 4-4.5, at which the hydrolysis of polyphenols of high molecular weight to those of low molecular weight is promoted.

It is known that oleuropein, one of the main polyphenols contained in OMWW, is hydrolyzed into elenolic acid and hydroxytyrosol (HT) (L.M. Panizzi, J.M. Scarpati, and E.G. Oriente, "Constitution of oleuropein, bitter and hypotensive action glycoside of olive". Note II. Gazz. Chim. Ital. 90:1449-1485, 1960).

The interest in hydroxytyrosol is also apparent from the scientific literature which reports various biological syntheses.

For example, Briante and co-workers (Briante, R.; Cara, F.L.; Tonziello, M.P.; Febbraio, F.; Nucci, R.; Antioxidant Activity of the Main Bioactive Derivatives from Oleuropein Hydrolysis by Hyperthermophilic β-Glycosidase; J. Agric. Food Chem. 2001, 49, 3198-3203; Briante, R.; Patumi, M.; Febbraio, F.; Nucci, R.; Production of highly purified hydroxytyrosol from Olea europaea leaf extract biotransformed by hyperthermophilic β-glycosidase; J. Biotechnol. 2004, 111, 67-77), reported works on the obtaining of HT from olive leaf extracts by enzymatic synthesis employing β-glucosidase immobilized on chitosan.

Khoufi and co-workers (Khoufi, S.; Hamza, M.; Sayadi, S.; Enzymatic hydrolysis of olive wastewater for hydroxytyrosol enrichment; Bioresour. Technol. 2011, 102, 9050-9058) used the olive mill waste waters as the raw material and proposed an enrichment method of hydroxytyrosol for the use, in the same olive mill waste waters, of the *Aspergillus Niger* microorganism in thermophilous conditions (50°C, 2 h).

A further method of enriching hydroxytyrosol for hydrolysis from oleuropein is described, for example, in the Italian patent application FI2006A000155, of CNR-ISE of Florence and Antico Frantoio Toscano in Bibbona, where the use of acids is reported, such as citric acid, to promote hydrolysis of oleuropein and inhibit polyphenoloxidase (PPO), an enzyme that oxidises the phenolic compounds to quinones thus making the water darker and increasing the biotoxicity of the wastewater.

GB2415136 discloses a process for obtaining the conversion of oleuropein into hydroxytyrosol from the byproducts of olive oil extraction by three successive steps. Specifically, in the first step, a first polar solvent is added to facilitate the removal of suspended solids by microfiltration or centrifugation, then enzymes (glucose oxidase and esterase) are added to hydrolyze oleuropein in hydroxytyrosol and finally a second, apolar solvent is added for the removal of oil; in the second step, a concentrate of polyphenols is obtained from the ultrafiltration permeate and from the nanofiltration retentate; finally, in the third step, said polyphenol concentrate is further concentrated by evaporation under vacuum. Villanova et al. (Villanova, L., Fasiello G., Merendino, A., in US20090023815, EP 1623960 and US 7427358, owned by LACHIFARMA srl), propose an OMWW treatment with sequential filtration (by reverse osmosis microfiltration) and subsequent chemical conversion of tyrosol to hydroxytyrosol by using a ruthenium trimethylene oxide in acidic conditions as heterogeneous catalyst.

Pizzichini and Russo in WO2005123603 describe the sequential use of microfiltration (MF), ultrafiltration (UF), nanofiltration (NF) and reverse osmosis (RO) for recovering the phenolic fraction and obtaining a complete OMWW purification.

Sequential filtration for recovering the phenolic fraction from the OMWW is also described in EP1870171 as specific steps of a more complex treatment method for recovery of the by-products of the oleaginous processing, which specifically provides for the combination of different filtrations with clear- flocculation with polyelectrolytes.

Fernandez-Bolanos et al. in US20056849770 describe an absorption process of phenols by ion exchange resins. Products manufactured by this process are marketed under the trade name Hytolive^{®}1 and Hytolive^{®}2.

Crea in US 7261909, US 2008009000, WO200218310 and WO 2004005228 describes a process wherein the hydroxytyrosol enrichment in olive mill waste waters is carried out by acid hydrolysis of the oleuropein by citric acid followed by extraction with supercritical CO₂.

Finally, processes for treating OMWW are also known, which are aimed at obtaining lyophilized polyphenolic mixtures.

For example, Jingren He et Al (Jingre He, Alister-Briggs M., De Lyster T., Jones G.P., "Stability and Antioxidant potential of purified olive mill wastewater extracts", Food Chemistry 131 (2012), 1312-1321) describe the preparation, characterization and properties of certain specific phenolic extracts obtained in the form of lyophilizates from OMWW, wherein said lyophilizates are prepared by a process comprising centrifugation, filtration, adsorption on amphoteric resins and elution with different solvents, followed by concentration by vacuum evaporation. Various methods of enrichment in polyphenols and phenols, and in particular in hydroxytyrosol, from olive mill waste waters have therefore been studied and carried out, as the raw material is advantageously low cost, still being an environmental pollutant to be disposed of. However, as clear from the above description of the enrichment methods, the processes and facilities described to date are very complicated and require high management and implementation costs.

The object of the present invention is therefore to provide a process for enriching the concentration of polyphenols and phenols, especially hydroxytyrosol, in olive mill waste waters (OMWW), which is at the same time simple to implement and cost-effective.

### SUMMARY

The inventors of the present invention have surprisingly found that olive mill waste waters (OMWW) could be enriched in polyphenols and phenols, preferably hydroxytyrosol, by means of a process involving the delivery of nitrogen in the form of liquid or gas, and keeping in contact said delivered nitrogen and the OMWW for at least a specific time period.

Advantageously, the process of the invention not only overcomes the drawbacks of the prior art since it provides a simple process, but it also allows obtaining concentrations in polyphenols and phenols that are higher than those so far expected by the known enrichment methods.

Therefore, the invention relates to a process for enriching the concentration in total phenols and polyphenols in olive mill waste waters, comprising the steps of:
(a) delivering at least one nitrogen flux, in the form of liquid or gas, in said olive mill waste waters, in an amount of 0.2-2 m³ nitrogen gas/m³ OMWW, wherein said nitrogen flux is delivered in amounts such that the percentage of oxygen dissolved in the olive mill waste waters at the end of said delivering step is lower than 0.7 mg/L;
(b) keeping said olive mill waste waters and said delivered nitrogen in contact for at least 7 days; and
(c) obtaining olive mill waste waters enriched in the concentration in said total phenols and polyphenols.

In the enrichment process of the present invention, nitrogen flux means a continuous jet of nitrogen which can be delivered simultaneously through one or more means, which are possibly arranged in different positions within the vessel containing the olive mill waste waters subjected to treatment.

It is thus understood that two nitrogenous jets, delivered discontinuously, i.e. delayed each other for a certain period of time, are considered as two different fluxes of nitrogen, for the purposes of the present invention.

In addition, in the present invention, when in step b) it is indicated that the contact between the olive mill waste waters and the nitrogen delivered therein is maintained for at least 7 days, this is intended to cover a period of time equal to or greater than 7 days.

In an advantageous aspect of the invention, said at least one nitrogen flux of step a) is delivered in amount such that the percentage of oxygen dissolved in the olive mill waste waters at the end of said delivery step a) is lower than 0.7 mg/L, preferably lower than 0.6 mg/L, even more preferably lower than 0.5 mg/L.

In a further advantageous aspect of the invention, said flux of nitrogen is in the form of gas.

The olive mill waste waters may also include solid fractions comprising olive pulp residues.

Advantageously, in a preferred embodiment, the process of the invention provides for grinding such suspended solids.

In another aspect, the invention further provides for adding to the olive mill waste waters, before step a) of delivering at least one flux of nitrogen, residual solid materials from olive processing, such as plant or olive parts, such as stones, leaves, olive branches, preferably olive leaves.

In a further advantageous aspect, the invention may provide for addition to the olive mill waste waters, containing the above mentioned solid residual materials, before step a) of delivering the at least one flux of nitrogen, of lactic bacteria, optionally previously isolated from the same olive mill waste waters.

### DESCRIPTION OF THE FIGURES

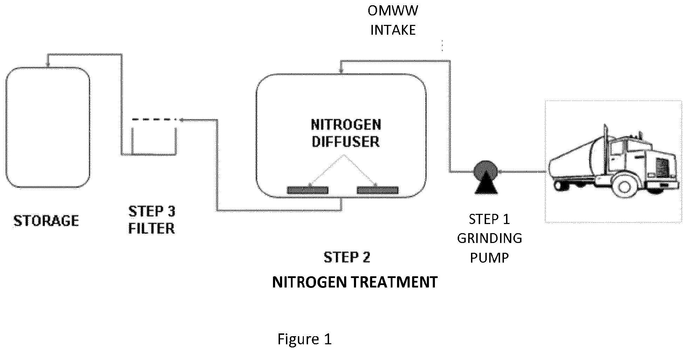

Figure 1 shows a scheme of the enrichment process of olive mill waste waters and relative storage.

### DETAILED DESCRIPTION OF THE INVENTION

The invention therefore relates to a process for enriching the concentration in total phenols and polyphenols in olive mill waste waters, comprising the steps of:
(a) delivering at least one nitrogen flux, in the form of liquid or gas, in said olive mill waste waters, in an amount of 0.2-2 m³ nitrogen gas/m³ OMWW, wherein said nitrogen flux is delivered in amounts such that the percentage of oxygen dissolved in the olive mill waste waters at the end of said delivering step is lower than 0.7 mg/L;
b) keeping said olive mill waste waters and said delivered nitrogen in contact for at least 7 days; and
(c) obtaining olive mill waste waters enriched in the concentration in said total phenols and polyphenols.

Step (a) is the delivering step of at least one nitrogen flux in olive mill waste waters (OMWW), said nitrogen flux being in the form of liquid or gas, preferably gas. Preferably, said nitrogen flux is delivered in an amount of 0.4-1.2 m³ nitrogen gas/m³ OMWW, even more preferably 0.5-1 m³ nitrogen gas/m³ OMWW.

The amount of delivered nitrogen is advantageously defined so as to ensure that the percentage of oxygen dissolved in the olive mill waste waters at the end of the delivering step a) is lower than 0.7 mg/L, preferably lower than 0.6 mg/L, even more preferably lower than 0.5 mg/L.

Advantageously, said step (a) of delivering at least one nitrogen flux in olive mill waste waters provides for delivering said nitrogen flux, in the form of gas or liquid, at the time of collecting the OMWW in the treatment tanks.

Step b) of keeping in contact said olive mill waste waters and said delivered nitrogen takes place for at least about 7 days. Keeping into contact is preferably carried out for a period of time of from about 7 days to about 28 days, more preferably from about 7 days to about 21 days, more preferably from about 7 to about 14 days.

Said contact step b) preferably takes place in a closed environment, preferably airtight.

In an advantageous embodiment, during or at the end of step b) of keeping in contact olive mill waste waters and delivered nitrogen, further nitrogen fluxes may be delivered before obtaining the olive mill waste waters enriched in one or more phenols and/or polyphenols of step c).

Therefore, after carrying out a first step a) of delivering at least one nitrogen flux and during or at the end of step b) of keeping in contact the olive mill waste waters and the delivered nitrogen for at least 7 days, the olive mill waste waters may be subjected to at least one further nitrogen flux delivery.

The further delivery of nitrogen fluxes, during or at the end of step b) of keeping in contact olive mill waste waters and delivered nitrogen, may be in the range from 1 to 4, preferably from 1 to 3, more preferably from 1 to 2, even more preferably 1, before obtaining the enriched olive mill waste waters of step c).

In a preferred embodiment, said further delivery of nitrogen fluxes is carried out, during or at the end of step b) of keeping in contact the OMWW and the delivered nitrogen, about every 7 days after the previous delivery, that is, the time between two successive deliveries of nitrogen fluxes is 7 days.

In a preferred embodiment of the invention, the process comprises a first stage a) of a first delivery of nitrogen flux and, during or at the end of the contact keeping period referred to in step b), further fluxes of nitrogen fluxes after about 7, about 14 and about 21 days after said first nitrogen delivery of step a), more preferably after about 7 and about 14 days from said step a), even more preferably a single subsequent delivery of nitrogen flux after about 7 days from the first delivery.

In a preferred embodiment, the process of the invention is carried out in tanks equipped with liquid or gas nitrogen delivery systems, preferably gas, arranged at the bottom. Said dispensers may preferably be tubular or disc-shaped, made of polymeric, steel or ceramic material.

In step (c) of obtaining olive mill waste waters enriched in the concentration in total phenols and polyphenols, preferably said enrichment is from about 20 to about 40% with respect to the initial concentration of at least one phenol and/or polyphenol. Specifically, in said step (c), the enrichment in hydroxytyrosol is from about 20 to about 200%, more preferably said enrichment is from about 30 to about 100%, even more preferably from about 50 to about 70% with respect to the initial concentration of hydroxytyrosol.

Advantageously, in said step (c), the enrichment in tyrosol is preferably from about 40 to about 140%, more preferably said enrichment is from about 80 to about 100% with respect to the initial concentration of said tyrosol.

In a further preferred embodiment of the invention, the OMWW, after treatment, can be filtered to remove suspended solids before their storage into storage tanks. Advantageously, the process according to the invention is carried out at room temperature.

Advantageously, the process according to the invention is carried out at a pressure within the range from 1 to about 1.2 bar, preferably from about 1 to about 1.1 bar. Olive mill waste waters (OMWW) of the process of the invention are waste waters resulting from the processing of olive oil and are obtained during the separation of water from the oil wort and from the plant washing operations.

Preferably, the OMWW enrichment process is carried out using the waters resulting from oil extraction processes, either discontinuous under pressure or continuous by two-phase or three-phase centrifugation.

Preferably, the OMWW used in the enrichment process have a pH of between 2-8, more preferably between 3-5, a content of suspended solids of between 10,000 - 100,000 mg/L, more preferably between 20,000-70,000 mg/L, a dry residue of between 1-12% by weight, more preferably of between 2-8%, a total phenol content (determined by the Folin Ciocalteau reaction method, expressed as mg gallic acid equivalents/L OMWW) of between 100-15,000, more preferably between 500-10,000, a hydroxytyrosol content (determined via HPLC-UV DAD) of between 25-4,000 mg/L, more preferably of between 100-2,500 mg/L.

Olive mill waste waters collected from mills may further comprise large fractions of solids, generally suspended and consisting of olive pulp residue partially ground during the pressing process.

Advantageously, the process of the invention therefore provides also for, in a preferred embodiment, carrying out a grinding of such suspended solids before before the step a) of treatment with nitrogen.

Said grinding step can be preferably carried out during the pumping of olive mill waste waters in the tank where the delivery of at least one nitrogen flux is carried out, by means of grinding pumps. Advantageously, the reduction of the size of these solids, generally suspended, allows to obtain solids having a size of from about 1 mm to about 0.5 mm, which favors the diffusion of the phenolic compounds contained therein, thus increasing the yield of enrichment in the concentration of phenols and polyphenols.

Advantageously, the olive mill waste waters at the end of the enrichment process can be filtered for the partial removal of suspended solids, with size generally above 0.25 mm, and then transferred to storage containers, possibly kept in an inert atmosphere. Filtration can be carried out through known filtration devices, such as drum, screw filters or filter presses. The suspended collected solids can then advantageously be returned to the treatment process with nitrogen.

In another aspect, the invention further provides for adding to the olive mill waste waters, before the nitrogen delivery step a), residual solid materials from olive processing, such as plant or olive parts, such as stones, leaves, olive branches, preferably olive leaves. The possible addition of said residual solid materials from processing in the enrichment step, advantageously, not only catalyzes the enrichment but allows the use of residues and waste that would otherwise be disposed of.

In a further advantageous aspect, the invention may provide for adding lactic bacteria to the olive mill waste waters, optionally containing also the above residual solid materials, before the treatment with nitrogen. Preferably, said lactic bacteria are of the genus *Lactobacillus.*

Olive mill waste waters treated with the process of the invention are enriched with at least one phenol and/or polyphenol, and particularly hydroxytyrosol, already after about one week of treatment, in particular in a time interval of from about 7 days to about 28 days, more preferably from about 7 days to about 21 days, even more preferably from about 7 to about 14 days, thereby advantageously increasing their concentration by more than 50, i.e. by about 15% more than the chemical processes based on acid hydrolysis using citric acid.

Moreover, the process with nitrogen is economically more advantageous than with other treatments, such as the one with citric acid described in the prior art. Typically, in fact, the dose of citric acid needed to lower the pH of OMWW from 4-4.5 to about 3 is 30 kg/m³ OMWW, with an approximate cost of 0.6 €/kg citric acid, i.e. a cost of 18 €/m³ OMWW.

Otherwise, nitrogen costs about 3.2 €/m³, thus by adding at most about 1 m³ per m³ OMWW/week for 3 weeks, the treatment cost is about 9.6 €/m³ OMWW, i.e. about half the treatment with citric acid.

The cost-effectiveness of the process of the invention is therefore clear.

Advantageously, the olive mill waste waters treated with nitrogen according to the invention and admixed with lactic bacteria, preferably admixed as inoculum and more preferably obtained from a consortium by isolation from the same olive mill waste waters, are enriched with hydroxytyrosol in just about 2 weeks, with an increase in the concentration of more than 50%.

The enrichment with hydroxytyrosol is therefore more effective in the embodiment that requires the presence of lactic bacteria.

In the even more advantageous embodiment, in which olive mill waste waters also contain olive leaves, are treated with nitrogen according to the process of invention and admixed with inoculum of a consortium of lactic bacteria, obtained preferably by isolation from the same olive mill waste waters, before the delivery step a), an enrichment of about 100% with hydroxytyrosol is obtained in only about 14 days. The advantageous properties of the process of the invention are also supported by the following experimental part.

### EXPERIMENTAL PART

### Example 1: Comparison of different treatment methods of olive mill waste waters.

Olive mill waste waters from the company Antico Frantoio Toscano, Bibbona (Livorno province) were subjected to either a treatment according to the process of invention, and to alternative treatments, in order to evaluate the enrichment obtained in the concentration of hydroxytyrosol (HT).

Specifically, the following table 3 shows the change over time of the concentration of hydroxytyrosol (expressed as mg/L) as a result of the following treatments conducted on samples of olive mill waste water as is (OMWW AS IS) and after microfiltration at 20 µm (MF OMWW):
- Anaerobic condition 1 (AN1-N2): delivery of nitrogen in the OMWW sample (process of the invention);
- Anaerobic condition 2 (AN2-CO2): delivery of CO2 in the OMWW sample (acid condition);
- Aerobic condition 1 (AE1-Air): delivery of air in the OMWW sample;
- Aerobic condition 2 (AE2-CA): delivery of air in the OMWW sample and maintaining of the pH value to 3 with citric acid (prior art process).

The gases used in the four comparative tests were delivered with a flux of 1 L/min for 1 minute per liter of OMWW at the beginning of treatment and then, after a step of keeping the sample in contact with the gas delivered for 7 days, two additional nitrogen fluxes were delivered, after 7 days from each other.

In other words, the flux of gas was delivered at the time of collection of the OMWW and then again after 7 and 14 days from that time.

Samples were taken and analyzed before the start of each delivery of gas flux and at the end of the observation period, i.e. upon collection of the OMWW (T0), after 7 days from the first gas delivery (T1), after 7 days from the second gas delivery (T2), and finally after 7 days from the third and final gas delivery, that is, at the end of the observation period (T3).

During the entire treatment, gases were therefore delivered three times, with delivery 7 days apart from each other, and the olive mill waste waters remained in contact with the gases delivered, in closed tanks, for at least 7 days and up to 21 days, for a total treatment period of 21 days.

**Table 3. HT concentration (mg/L) in OMWW as is (OMWW AS IS) and in microfiltered OMWW (MF OMWW) in the different treatment conditions.**

| | **Treatment** | **T0** | **T1** | **T2** | **T3** |
|---|---|---|---|---|---|
| **OMWW AS IS** | **AN1** - **N2** | 601.1 | 685.4 | 815.0 | 916.3 |
| | **AN2 - CO2** | | 734.6 | 765.4 | 775.9 |
| | **AE1** - **Air** | | 702.4 | 760.3 | 738.8 |
| | **AE2** - **CA** | | 800.7 | 798.7 | 799.7 |
| **MF OMWW** | **AN1 - N2** | 565.3 | 625.9 | 615.8 | 620.6 |
| | **AN2 - CO2** | | 597.6 | 580.0 | 597.1 |
| | **AE1 - Air** | | 589.8 | 567.6 | 577.7 |
| | **AE2** - **CA** | | 653.5 | 625.0 | 637.6 |

The results in table 3 indicate that:
- the treatments carried out on OMWW AS IS led to significantly higher enrichments in hydroxytyrosol than the enrichment obtained in the case of MF OMWW;
- treatment of OMWW AS IS with the prior art process, comprising the delivery of air and the use of citric acid (test AE2-CA) produced an enrichment in hydroxytyrosol after only 7 days of treatment, higher than the enrichment obtained in the other treatment conditions with the exception of the treatment according to the process of the invention;
- with the treatment according to the process of the invention, the use of nitrogen allowed a continuous increase in the concentration of hydroxytyrosol, which reached after 21 days of treatment the maximum concentration values obtained in the various tests, with an enrichment in hydroxytyrosol higher than 52.4%;
- treatments using air fluxes and CO₂ were the less efficient.

The carried out experiments therefore show incredibly higher results in terms of enrichment of the olive mill waste waters in hydroxytyrosol, when treated according to the process of the invention and therefore subjected to nitrogen fluxes, both with respect to the prior art treatment that combines CO₂ fluxes with the addition of citric acid, and with respect to the further alternative treatments tested, in which only gases, air and CO₂ were delivered.

In particular, already after 2 gas delivering steps, i.e. already after 14 days of treatment (concentration of hydroxytyrosol at T2), the treatment according to the process of invention, simple and cost-effective, equaled and exceeded the result obtained according to the more expensive and complicated prior art process, and after 21 days, said treatment according to the process of the invention was able to generate an enrichment significantly higher than any other method tested.

This test shows that the presence of a fraction of suspended solids in the OMWW has an advantageous effect on the hydroxytyrosol enrichment process in olive mill waste waters; in fact, once these solids have been almost completely removed by microfiltration, the same increase in the HT concentration was not observed.

### Example 2: Treatment of further OMWW according to the process of the invention.

In order to verify the effectiveness and reproducibility of the treatment of the invention, an experiment was also conducted on olive mill waste waters from a different source, in particular coming from the company Frantoio Sociale Monti Pisani di Vicopisano (Province of Pisa).

As in the case of the experiments described in Example 1 above, said olive mill waste waters were subjected, at room temperature, in closed containers, to delivery of a nitrogen gas flux equal to 1 L/min for 1 minute per liter of OMWW at the beginning of treatment and then, after a step of keeping the sample in contact with the gas delivered for 7 days, 2 additional nitrogen fluxes were delivered, after 7 days from each other.

In other words, the flux of gas was delivered at the time of collection of the OMWW and then again after 7 and 14 days from that time.

Samples were taken and analyzed before the start of each delivery of gas flux and at the end of the observation period, i.e. upon collection of the OMWW (T0), after 7 days from the first gas delivery (T1), after 7 days from the second gas delivery (T2), and finally after 7 days from the third and final gas delivery, that is, at the end of the observation period (T3).

In particular, the values of concentration (expressed in mg/L) of hydroxytyrosol (HT) and tyrosol (TY), shown in the following table 4, were determined.

**Table 4. HT (mg/L) and TY (mg/L) concentrations in OMWW treated with the process according to the invention.**

| | **T0** | **T1** | **T2** | **T3** |
|---|---|---|---|---|
| HT* (mg/L) | 258.36 | 354.61 | 544.22 | 542.39 |
| TY* (mg/L) | 45.81 | 93.63 | 96.72 | 109.86 |

| | | | | |
|---|---|---|---|---|
| *HPLC method | | | | |

As is clear from the data in the table, even also in this second experiment, with the process of the invention, it was possible to obtain a surprising enrichment both in hydroxytyrosol (HT) and in tyrosol (TY), which are the most representative phenolic components of the OMWW matrix.

In particular, already after the second week, both the concentration of hydroxytyrosol and tyrosol in the olive mill waste waters had increased by about 111%; even the concentration of tyrosol showed a significant increase at the end of the third week, with an enrichment of 140%.

### Example 3: Comparative enrichment tests carried out in different conditions according to the process of invention.

Olive mill waste waters from Cooperative Montalbano olio e vino di Vinci (Province of Florence) were subjected to the process of invention.

In particular, according to step a) of the process of the invention, a flux of 600 L of nitrogen gas was delivered by means of cylindrical dispensers with polymeric membrane in 1 m³ of olive mill waste waters contained in a closed tank.

After 7, 14, 21 and 28 days from said first delivery of nitrogen, 600 L of nitrogen fluxes were further delivered. Specifically, a flux of 600 L of nitrogen was delivered after 7 days from the first delivery, a further flux of 600 L of nitrogen was delivered after 14 days from the first delivery, a still further flux of 600 L of nitrogen was delivered after 21 days from the first delivery and a last further flux of 600 L of nitrogen was delivered after 28 days from the first delivery, for a total of 4 further delivery of nitrogen of 600 L each, delivered after the first delivery step a).

The whole process was conducted at room temperature.

Samples of said olive mill waste waters were collected and analyzed before the beginning of the treatment, and then 1, 2, 3, 4 and 5 weeks after starting the treatment, i.e. before each repetition of delivery of the gas flux, and finally 7 days after the fourth and final gas delivery, determining the concentration (expressed as mg/L) of both hydroxytyrosol (HT) and tyrosol (TY).

Samples of said treated water were also taken in the 2 weeks thereafter, to observe any "tail" effects of further enrichment of the same.

The purpose of the experiment was to identify the optimal conditions to carry out the process of the invention.

The results obtained are shown in the following Table 5.

**Table 5. HT (mg/L) and TY (mg/L) concentrations in OMWW treated according to the process of the invention during the 7 weeks of observation.**

| | **T0** | **T1** | **T2** | **T3** | **T4** | **T5** | **T6** | **T7** |
|---|---|---|---|---|---|---|---|---|
| **HT** | 543.64 | 614.46 | 812.97 | 796.53 | 811.59 | 803.66 | 789.65 | 813.10 |
| **TY** | 93.05 | 105.31 | 130.32 | 125.48 | 130.53 | 140.03 | 132.81 | 136.57 |

As can be seen from the results shown in Table 5, the enrichment in phenolic compounds was completed already after the second week from the first delivery of the nitrogen flux, i.e. after delivering two nitrogen fluxes, each of 0.6 m³, delivered one week from each other, with a total consumption of nitrogen of 1.2 m³ (that is, 1.2 m³/m³ OMWW).

The concentrations of phenolic compounds in fact remained almost unchanged in the following weeks.

No particular "tail effect" was observed in the enrichment of OMWW in the weeks after the last delivery of the nitrogen flux, in which there was an absence of further delivery (weeks 6 and 7, concentration values in HT and TY at T6 and T7). Optimal conditions for carrying out the process of the invention can therefore be identified in a process that involves the repetition of delivering a flux of nitrogen gas, after 7 days of the first delivery of nitrogen of step a), and the collection of the enriched olive mill waste waters of step c) of the invention, after about 14 days from the date of execution of said step a).

The results obtained also show that the process of the invention allows not only enriching the olive mill waste waters in polyphenolic compounds, but also stabilizing them with respect to the content of said polyphenols over time.

### Example 4: Effect of adding lactic bacteria to the process according to the invention.

Some strains of lactic bacteria were isolated from the OMWW to produce a mixture of strains to be used as inoculum into the same OMWW, freshly collected, to verify their possible effect on the enrichment of hydroxytyrosol.

Starting from "mature" OMWW, with the specific indigenous microbial flora fully developed, serial dilutions and subsequent cultures on selective medium MRS Agar were set up in order to select and isolate any lactic bacteria eventually present.

Microorganisms grown on first isolation plates were then selected based on the morphology and the appearance of colonies and sub-cultured through several steps on MRS Agar plates to obtain their isolation in pure culture.

Pure strains were then frozen at -70 °C until their use in order to keep their characteristics unchanged. The three isolated strains were then used to conduct tests 1-3 described below.

### TEST 1: test of comparison of the HT enrichment activity of different strains of lactic bacteria isolated from OMWW.

The ability of the strains to enrich demineralized water in HT, used as inoculum vehicle containing chopped olive leaves (as known containing various phenols, including oleuropein), was first tested.

About 150 g of leaves were chopped into 250 mL of demineralized water.

About 150 g of leaves were homogenized by a mixer in 250 mL of demineralized water. 35 g of the resulting mixture (having a dry residue of 17.4%) were dispersed in 70 mL of saline solution containing 2 mL of inoculum (about 3% by volume with a concentration of 10⁸ cfu/mL) consisting of:
- Consortium of the three strains (1)
- Strain 1 alone (2)
- Strain 2 alone (3)
- Strain 3 alone (4)

A test with only demineralized water without inoculum was inserted as blank (0) by comparison.

The dispersions produced were stored at 20 °C in an incubator, then after 1 hour and several days, 5 mL of supernatant (replenished each time with demineralized water) were collected for the determination of HT. The results are shown in Table 6.

**Table 6. HT enrichment of the dispersion of olive leaves.**

| **Treatment** | **No.** | **HT concentration (mg/L)** | | | |
|---|---|---|---|---|---|
| | | **1 hour** | **3 days** | **7 days** | **18 days** |
| Blank | 0 | 85.67 | 215.95 | 217.01 | 215.21 |
| Consortium | 1 | 93.51 | 359.42 | 403.32 | 458.60 |
| Strain 1 | 2 | 88.54 | 323.00 | 387.08 | 358.46 |
| Strain 2 | 3 | 79.62 | 292.86 | 303.97 | 294.09 |
| Strain 3 | 4 | 85.61 | 350.37 | 366.34 | 375.67 |

This preliminary test showed the effect of strains to enrich the supernatant solution with HT. Among the various solution tested, the consortium of strains showed increased activity compared to the individual strains and was therefore chosen as a bacterial matrix to be used within the subsequent trials, to support the enrichment process of the invention.

### TEST 2: HT enrichment test of fresh OMWW by the addition of the microbial consortium before the nitrogen treatment step (a).

This test used the fresh OMWW of example 3, making a single delivery of nitrogen flux on the OMWW collected, using cylindrical diffusers with polymeric membrane, which delivered 600 L/m³ of OMWW (step a)); the step b) of keeping in contact was continued for three weeks.

For this test, only the microbial consortium that the previous test had shown to be the most promising (in amounts of about 3% by volume in OMWW with concentration of 10⁸ cfu/mL) was used. The blank was represented by OMWW without inoculum.

The following Table 7 shows the results of applying the process according to the invention to such OMWW, in the presence and in the absence of inoculum of lactic bacteria.

Samples were collected and analyzed before the delivery of nitrogen (T0) and then 1, 2 and 3 weeks (T1, T2 and T3) after said delivery.

**Table 7. HT concentration values in the OMWW in the different experimental conditions tested.**

| **Treatment** | **No.** | **HT concentration (mg/L)** | | | |
|---|---|---|---|---|---|
| | | **T0** | **T1** | **T2** | **T3** |
| Blank | 0 | 543.64 | 514.46 | 812.97 | 796.53 |
| Consortium | 1 | 552.83 | 784.32 | 822.64 | 818.82 |

The results show that due to the use of the inoculum of lactic bacteria, already one week after the delivery of the nitrogen gas flux, the enrichment in HT is almost complete. The process according to the invention therefore, with the use of lactic bacteria, seems to have greater effectiveness of enrichment in a shorter time.

### TEST 3: HT enrichment test of fresh OMWW by the addition of the microbial consortium and chopped olive leaves before the nitrogen delivering step (a).

In this test, the same test conditions above were used, adding in addition the mixture of olive leaves in a leaf/OMWW ratio of 1:2. The blank is the OMWW without inoculum and without olive leaves.

The following Table 8 shows the results of applying the process according to the invention to such OMWW, in the presence and in the absence of inoculum of lactic bacteria and olive leaves. Samples were collected and analyzed before the beginning of the treatment (T0) and then 1, 2 and 3 weeks (T1, T2 and T3) after the delivery of the nitrogen flux.

**Table 8. HT concentration values in the OMWW in the different experimental conditions tested.**

| **Treatment** | **No.** | **HT concentration (mg/L)** | | | |
|---|---|---|---|---|---|
| | | **T0** | **T1** | **T2** | **T3** |
| Blank | 0 | 543.64 | 876.31 | 891.12 | 902.32 |
| Consortium and olive leaves | 1 | 561.44 | 1120.51 | 1297.41 | 1303.27 |

The results show that due to the simultaneous use of the lactic bacteria and olive leaves inoculum, an extraordinary increase in the content of hydroxytyrosol can be obtained and also in this case, after just two weeks of the delivery of nitrogen the enrichment is complete.

### Example 5: Experimental tests conducted on a pilot plant for the treatment of OMWW according to the process of invention.

A pilot plant was implemented according to the scheme in figure 1.

The olive mill waste waters from the Cooperative Montalbano olio e vino di Vinci (Province of Florence) were collected with a tanker truck, thereafter by suction by a submersible grinding pump, introduced into four closed treatment reactors consisting of tanks containing 1 m³ of OMWW each, equipped with EPDM rubber cylindrical bubbling systems to dispense the nitrogen flux.

Thereafter, they were subjected, with the exception of tank 1 representing the untreated reference sample, to delivery of one or more fluxes of 60 L/min nitrogen, 1 bar pressure, of 9 minutes each, according to the experimental plan shown in the following Table 9.

Throughout the treatment period, the oxygen content dissolved in the OMWW contained in tanks 2, 3 and 4 was always below 0.5 mg/L.

**Table 9. Experimental treatment plan of OMWW in the four different tanks.**

| | Tank 1 | Tank 2 | Tank 3 | Tank 4 |
|---|---|---|---|---|
| Nitrogen gas delivering scheme | No delivery | Nitrogen delivery: | Nitrogen delivery: | Nitrogen delivery: |
| | | - upon collection (step a); | - upon collection (step a); | - only upon collection (step a). |
| | | - after 1 week; | - after 1 week. | |
| | | - after 2 weeks. | | |
| Total nitrogen volume (L) entered in each tank | 0 | 1620 | 1080 | 540 |

Samples of the olive mill waste waters contained in the four tanks were collected and analyzed at the time of collection before delivering the first nitrogen flux (T0), and then weekly (T1, T2 and T3) before the further delivery of nitrogen according to the treatment scheme set out in Table 9.

Specifically, the timing of collection of each sample therefore were:
T0: at the time of collection and before delivering the first nitrogen flux;
T1: 7 days after the first delivery of nitrogen and before the second delivery of the nitrogen flux (if provided by the treatment scheme in Table 9);
T2: 14 days after the first delivery of nitrogen and before the third delivery of the nitrogen flux (if provided by the treatment scheme in Table 9);
T3: 21 days after the first delivery of nitrogen.

In the taken samples the values of concentration (mg/L) of hydroxytyrosol (HT), tyrosol (TY) and total phenols and polyphenols (PF) were determined in accordance with the Folin Ciocalteau reaction method, expressed as mg Gallic acid equivalents/L OMWW. All the values are reported in Table 10.

At the end of the treatment, the OMWW obtained are then filtered by a steel mesh sieve having pore size of 500 microns, for the removal of the rough suspended particulate and stored in a collecting tank.

**Table 10. Concentrations of total polyphenols (PF), hydroxytyrosol (HT) and tyrosol (TY) in the OMWW in the different experimental conditions tested.**

| Parameters | Tank 1 | | | | Tank 2 | | | |
|---|---|---|---|---|---|---|---|---|
| Times | T0 | T1 | T2 | T3 | T0 | T1 | T2 | T3 |
| PF(mg/L) | 2593 | 2604 | 2592 | 2598 | 2576 | 3189 | 3369 | 3348 |
| HT(mg/L) | 280.7 | 297.2 | 288.6 | 293.4 | 274.1 | 744.3 | 827.1 | 814.6 |
| TY(mg/L) | 45.6 | 48.4 | 39.4 | 43.7 | 40.4 | 73.7 | 83.3 | 81.6 |

| Parameters | Tank 3 | | | | Tank 4 | | | |
|---|---|---|---|---|---|---|---|---|
| Times | T0 | T1 | T2 | T3 | T0 | T1 | T2 | T3 |
| PF(mg/L) | 2614 | 3235 | 3298 | 3324 | 2589 | 3251 | 3294 | 3315 |
| HT(mg/L) | 299.1 | 782.9 | 804.3 | 821.5 | 285.9 | 786.4 | 792.2 | 809.3 |
| TY(mg/L) | 51.3 | 78.6 | 83.7 | 81.2 | 41.8 | 69.4 | 81.7 | 83.6 |

From the data obtained in the pilot testing, the efficacy of the treatment of the invention is evident. In fact, compared to the untreated OMWW of tank 1, the treated OMWW of tanks 2, 3 and 4 showed a marked increase in the concentration of phenols and polyphenols, hydroxytyrosol and tyrosol.

The maximum increase in the concentration in the various components object of observation was 1 week after the first delivery, with an enrichment that continued even after two weeks from the first delivery, stabilizing around the third week after the first delivery.

From the results of the experiments, there were also no significant differences in the final concentration values of PF, HT and TY obtained in tanks 2, 3 and 4 at the end of the 3 weeks of observation.

This suggests that the treatment methods with nitrogen could be optimized in terms of gas consumption by applying 0.5-0.6 m³ of Nitrogen gas/m³ OMWW even only at the time of harvesting the olive mill waste waters and leaving the OMWW and Nitrogen in contact within closed tanks for a period of time of about two to about three weeks, so that the reactions leading to the various enrichment may take place and complete.

Ultimately, all of the experimental data above indisputably shows that the process of the invention allows a significant enrichment of the olive mill waste waters in phenolic components of great interest, through an extremely simple and economical process, with very high yields in very short time.

This process can therefore be effectively applied to the olive mill waste waters, allowing their valuable use, alternative to disposal, as a concentrated source of phenolic compounds or, as such, in those industries and facilities where such enriched waters would be revealed directly usable within specific processing cycles.

## Claims

1. A process for enriching the concentration in total phenols and polyphenols in olive mill waste waters (OMWW), comprising the steps of:
(a) delivering at least one nitrogen flux, in the form of liquid or gas, in said olive mill waste waters, in an amount of 0.2-2 m³ nitrogen gas/m³ OMWW, wherein said nitrogen flux is delivered in amounts such that the percentage of oxygen dissolved in the olive mill waste waters at the end of said delivering step is lower than 0.7 mg/L;
(b) keeping said olive mill waste waters and said delivered nitrogen in contact for at least 7 days; and
(c) obtaining olive mill waste waters enriched in the concentration in said total phenols and polyphenols.

2. The process according to claim 1 , wherein said nitrogen flux of step a) is delivered in amounts such that the percentage of oxygen dissolved in the olive mill waste waters at the end of said delivering step is lower than 0.6 mg/L, even more preferably lower than 0.5 mg/L.

3. The process according to claim 1 or 2, where said nitrogen flux of step a) is in the form of gas, preferably in an amount of 0.4-1.2 m³ nitrogen gas/m³ OMWW, even more preferably 0.5-1 m³ nitrogen gas/m³ OMWW.

4. The process according to any one of claims 1 to 3, wherein step b) of keeping said olive mill waste waters and said delivered nitrogen in contact is carried out for a time period from 7 days to 28 days, more preferably from 7 days to 21 days, even more preferably from 7 to 14 days.

5. The process according to any one of claims 1 to 4, wherein said contact step b) takes place in a closed environment, preferably airtight.

6. The process according to any one of claims 1 to 5, wherein during or at the end of step b) of keeping in contact olive mill waste waters and delivered nitrogen, further delivery of nitrogen fluxes is carried out before obtaining the olive mill waste waters enriched in total phenols and polyphenols of step c).

7. The process according to claim 6, wherein said further delivery of nitrogen fluxes is in number from 1 to 4, preferably from 1 to 3, more preferably from 1 to 2, even more preferably 1.

8. The process according to claim 6 or 7 wherein said further delivery of nitrogen fluxes is carried out after 7, 14 and 21 days, preferably after 7 and 14 days, more preferably after 7 days, from the delivering step a).

9. The process according to any one of claims 1 to 8, wherein before step (a) of delivering at least one flux of nitrogen, a grinding step of the solids fraction present in the olive mill waste waters is carried out.

10. The process according to any one of claims 1 to 9, wherein before step (a) of delivering at least one flux of nitrogen, a step of addition of olive processing residual solids to the olive mill waste waters is carried out.

11. The process according to any one of claims 1 to 10, wherein before step (a) of delivering at least one flux of nitrogen, a step of adding lactic bacteria, preferably of the *Lactobacillus* species, to the olive mill waste waters, more preferably previously isolated from the same olive mill waste waters, is carried out.

12. The process according to claim 11, wherein said bacteria are present in a concentration of at least about 10⁸ UFC/mL.

13. The process according to any one of claims 1 to 12, wherein the OMWW of step (c) have an enrichment in the concentration of hydroxytyrosol from about 20 to about 200%, preferably from about 30 to about 100%, more preferably from about 50 to about 70% compared to the initial concentration of hydroxytyrosol.

14. The process according to any one of claims 1 to 13 wherein said process is carried out at room temperature, preferably at a pressure in the range of 1 to 1.2 bar, preferably 1 to 1.1 bar.

15. The process according to any one of claims 1 to 14, wherein a flux of nitrogen gas is delivered in step a) in an amount of 0.5 to 0.6 m³ of nitrogen gas/m³ of OMWW and step b) has a duration of 14 to 21 days.

## Patentansprüche

1. Verfahren zur Anreicherung der Konzentration an Gesamtphenolen und Polyphenolen in Olivenmühlenabwässern (OMWW), das die folgenden Schritte umfasst:
(a) Zuführen mindestens eines Stickstoffflusses in Form von Flüssigkeit oder Gas in die Olivenmühlenabwässer in einer Menge von 0,2-2 m³ Stickstoffgas/m³ von OMWW, wobei der Stickstofffluss in solchen Mengen zugeführt wird, dass der Gehalt des in den Olivenmühlenabwässern gelösten Sauerstoffs am Ende des Zuführungsschritts weniger als 0,7 mg/L beträgt;
(b) Halten die Olivenmühlenabwässer und den gelieferten Stickstoff mindestens 7 Tage lang in Kontakt; und
(c) Gewinnen die Olivenmühlenabwässer, die in der Konzentration an Gesamtphenolen und Polyphenolen angereichert sind.

2. Verfahren nach Anspruch 1, wobei der Stickstofffluss aus Schritt a) in solchen Mengen zugeführt wird, dass der Gehalt des in den Olivenmühlenabwässern gelösten Sauerstoffs am Ende des Zuführungsschritts weniger als 0,6 mg/L, noch bevorzugter weniger als 0,5 mg/L beträgt.

3. Verfahren nach Anspruch 1 oder 2, wobei der Stickstofffluss von Schritt a) in Form von Gas vorliegt, vorzugsweise in einer Menge von 0,4-1,2 m³ Stickstoffgas/m³ von OMWW, noch bevorzugter 0,5-1 m³ Stickstoffgas/m³ von OMWW.

4. Verfahren nach einem der Ansprüche 1 bis 3, wobei Schritt b) zum Halten die Olivenmühlenabwässer und den zugeführten Stickstoff in Kontakt über einen Zeitraum von 7 bis 28 Tagen, vorzugsweise von 7 bis 21 Tagen und noch bevorzugter von 7 bis 14 Tagen durchgeführt wird.

5. Verfahren nach einem der Ansprüche 1 bis 4, wobei der Kontaktschritt b) in einer geschlossenen, vorzugsweise luftdichten Umgebung stattfindet.

6. Verfahren nach einem der Ansprüche 1 bis 5, wobei während oder am Ende von Schritt b) zum Halten die Olivenmühlenabwässer und den zugeführten Stickstoff in Kontakt, eine weitere Zuführung von Stickstoffflüssen durchgeführt wird, bevor die an Gesamtphenolen und Polyphenolen angereicherten Olivenmühlenabwässer von Schritt c) erhalten werden.

7. Verfahren nach Anspruch 6, wobei die weitere Zuführung von Stickstoffflüssen eine Anzahl von 1 bis 4, vorzugsweise 1 bis 3, noch bevorzugter 1 bis 2, noch bevorzugter 1 ist.

8. Verfahren nach Anspruch 6 oder 7, wobei die weitere Zuführung von Stickstoffflüssen nach 7, 14 und 21 Tagen, vorzugsweise nach 7 und 14 Tagen, bevorzugter nach 7 Tagen, ab dem Zuführungsschritt a) erfolgt.

9. Verfahren nach einem der Ansprüche 1 bis 8, **dadurch gekennzeichnet, dass** vor dem Schritt (a) zum Zuführen mindestens eines Stickstoffflusses ein Zerkleinerungsschritt der in den Olivenmühlenabwässern vorhandenen Feststofffraktion durchgeführt wird.

10. Verfahren nach einem der Ansprüche 1 bis 9, wobei vor dem Schritt (a) zum Zuführen mindestens eines Stickstoffflusses ein Schritt der Zugabe von Restfeststoffen aus der Olivenverarbeitung zu den Olivenmühlenabwässern durchgeführt wird.

11. Verfahren nach einem der Ansprüche 1 bis 10, wobei vor dem Schritt (a) zum Zuführen mindestens eines Stickstoffflusses ein Schritt der Zugabe von Milchsäurebakterien, vorzugsweise der Art *Lactobacillus* zu den Olivenmühlenabwässern, die vorzugsweise zuvor aus denselben Olivenmühlenabwässern isoliert wurden, durchgeführt wird.

12. Verfahren nach Anspruch 11, wobei die Bakterien in einer Konzentration von mindestens etwa 10⁸ UFC/ml vorhanden sind.

13. Verfahren nach einem der Ansprüche 1 bis 12, wobei die OMWW aus Schritt (c) eine Anreicherung der Hydroxytyrosol-Konzentration von etwa 20 bis etwa 200 %, vorzugsweise von etwa 30 bis etwa 100 %, noch bevorzugter von etwa 50 bis etwa 70 % im Vergleich zur ursprünglichen Hydroxytyrosol-Konzentration aufweisen.

14. Verfahren nach einem der Ansprüche 1 bis 13, wobei das Verfahren bei Raumtemperatur, vorzugsweise bei einem Druck im Bereich von 1 bis 1,2 bar, vorzugsweise von 1 bis 1,1 bar, durchgeführt wird.

15. Verfahren nach einem der Ansprüche 1 bis 14, wobei in Schritt a) ein Stickstoffgasfluss in einer Menge von 0,5 bis 0,6 m³ von Stickstoffgas/m³ von OMWW zugeführt wird und Schritt b) eine Dauer von 14 bis 21 Tagen hat.

## Revendications

1. Procédé d'enrichissement de la concentration en phénols totaux et en polyphénols dans des eaux usées du moulin à huile (OMW), comprenant les étapes suivantes :
(a) délivrer au moins un flux d'azote, sous forme de liquide ou de gaz, dans lesdites eaux usées du moulin à huile, dans une quantité de 0,2-2 m³ de gaz d'azote/m³ d'OMW, dans lequel ledit flux d'azote est délivré dans des quantités telles que le pourcentage d'oxygène dissous dans les eaux usées du moulin à huile à la fin de ladite étape de délivrance est inférieur à 0,7 mg/L ;
(b) maintenir en contact lesdites eaux usées du moulin à huile et ledit azote délivré pendant au moins 7 jours ; et
(c) obtenir des eaux usées du moulin à huile enrichies en concentration desdits phénols et polyphénols totaux.

2. Procédé selon la revendication 1, dans lequel ledit flux d'azote de l'étape a) est délivré en quantités telles que le pourcentage d'oxygène dissous dans les eaux usées du moulin à huile à la fin de ladite étape de délivrance est inférieur à 0,6 mg/L, encore plus préférablement inférieur à 0,5 mg/L.

3. Procédé selon la revendication 1 ou 2, dans lequel ledit flux d'azote de l'étape a) est sous forme de gaz, de préférence dans une quantité de 0,4-1,2 m³ d'azote gazeux/m³ d'OMW, encore plus préférablement 0,5-1 m³ d'azote gazeux/m³ d'OMW.

4. Procédé selon l'une quelconque des revendications 1 à 3, dans lequel l'étape b) de maintien en contact desdites eaux usées du moulin à huile et dudit azote délivré est réalisée pendant une période de temps allant de 7 jours à 28 jours, plus préférablement de 7 jours à 21 jours, encore plus préférablement de 7 à 14 jours.

5. Procédé selon l'une quelconque des revendications 1 à 4, dans lequel ladite étape b) de mise en contact a lieu dans un environnement fermé, de préférence hermétique.

6. Procédé selon l'une quelconque des revendications 1 à 5, dans lequel, pendant ou à la fin de l'étape b) de maintien en contact des eaux usées du moulin à huile et de l'azote délivré, une délivrance supplémentaire de flux d'azote est effectuée avant l'obtention des eaux usées du moulin à huile enrichies en phénols et polyphénols totaux de l'étape c).

7. Procédé selon la revendication 6, dans lequel ladite délivrance supplémentaire de flux d'azote est en nombre de 1 à 4, de préférence de 1 à 3, plus préférablement de 1 à 2, encore plus préférablement 1.

8. Procédé selon la revendication 6 ou 7, dans lequel ladite délivrance supplémentaire de flux d'azote est effectuée après 7, 14 et 21 jours, de préférence après 7 et 14 jours, plus préférablement après 7 jours, à partir de l'étape de délivrance a).

9. Procédé selon l'une quelconque des revendications 1 à 8, dans lequel avant l'étape (a) de délivrance d'au moins un flux d'azote, une étape de broyage de la fraction de matières solides présente dans les eaux usées du moulin à huile est réalisée.

10. Procédé selon l'une quelconque des revendications 1 à 9, dans lequel avant l'étape (a) de délivrance d'au moins un flux d'azote, une étape d'ajout de résidus solides de traitement d'huile aux eaux usées du moulin à huile est réalisée.

11. Procédé selon l'une quelconque des revendications 1 à 10, dans lequel avant l'étape (a) de délivrance d'au moins un flux d'azote, une étape d'ajout de bactéries lactiques, de préférence de l'espèce *Lactobacillus*, aux eaux usées du moulin à huile, plus préférablement préalablement isolées des mêmes eaux usées du moulin à huile est réalisée.

12. Procédé selon la revendication 11, dans lequel lesdites bactéries sont présentes à une concentration d'au moins environ 10⁸ UFC/mL.

13. Procédé selon l'une quelconque des revendications 1 à 12, dans lequel les OMW de l'étape (c) présentent un enrichissement en concentration d'hydroxytyrosol d'environ 20 à environ 200 %, de préférence d'environ 30 à environ 100 %, plus préférablement d'environ 50 à environ 70 % par rapport à la concentration initiale d'hydroxytyrosol.

14. Procédé selon l'une quelconque des revendications 1 à 13, dans lequel ledit procédé est réalisé à température ambiante, de préférence à une pression comprise entre 1 et 1,2 bar, de préférence entre 1 et 1,1 bar.

15. Procédé selon l'une quelconque des revendications 1 à 14, dans lequel un flux d'azote gazeux est délivré à l'étape a) en une quantité de 0,5 à 0,6 m³ d'azote gazeux/m³ d'OMW et l'étape b) a une durée de 14 à 21 jours.
